# EUROPEAN PATENT APPLICATION

(11) **EP 2 641 971 A1**
(43) Date of publication of application: **25.09.2013**
(21) Application number: 13166906.1
(22) Date of filing: 29.01.2008
(51) Int. Cl.: C12N 15/113, C07H 21/00

(54) **Compounds and methods for modulating protein expression**

(30) Priority: 29.01.2007 US 887119 P
(62) Divisional of application: 08714113.1
(71) Applicant: Isis Pharmaceuticals, Inc., Carlsbad, CA 92010 (US)
(72) Inventor: Bhat, Balkrishen, Carlsbad, CA 92009 (US); Swayze, Eric E., Carlsbad, CA 92009 (US); Lima, Walter F., San Diego, CA 92014 (US); Crooke, Stanley T., Carlsbad, CA 92009 (US)
(74) Representative: Carpmaels & Ransford LLP

(57) **Abstract**

The present invention includes compositions and methods useful for modulating protein expression. In certain embodiments, the present invention includes oligomeric compounds comprising modified nucleosides and modified internucleoside linkages.

SEQUENCE LISTING

<110> Isis Pharmaceuticals, Inc.
      Balkrishen Bhat
      Eric E. Swayze
      Walter F. Lima
      Stanley T. Crooke

<120> COMPOUNDS AND METHODS FOR MODULATING
      PROTEIN EXPRESSION

<130> CORE0073WO

<150> 60/887,119
<151> 2007-01-29

<160> 4

<170> FastSEQ for Windows Version 4.0

<210> 1
<211> 19
<212> RNA
<213> Artificial Sequence

<220>
<223> Synthetic oligonucleotide

<220>
<221> misc_feature
<222> 1-19
<223> n = A,U,C or G

<400> 1
nnnnnnnnnn nnnnnnnnn                                                    19

<210> 2
<211> 20
<212> RNA
<213> Artificial Sequence

<220>
<223> Synthetic oligonucleotide

<220>
<221> misc_feature
<222> 1-20
<223> n = A,U,C or G

<400> 2
nnnnnnnnnn nnnnnnnnnn                                                   20

<210> 3
<211> 21
<212> RNA
<213> Artificial Sequence

<220>
<223> Synthetic oligonucleotide

<220>
<221> misc_feature

<222> 1-21
<223> n = A,U,C or G

<400> 3
nnnnnnnnnn nnnnnnnnnn n                                                21

<210> 4
<211> 19
<212> RNA
<213> Artificial Sequence

<220>
<223> Synthetic oligonucleotide

<400> 4
uugucucugg uccuuacuu                                                   19

## Description

### Sequence Listing

The present application is being filed along with a Sequence Listing in electronic format. The Sequence Listing is provided as a file entitled CORE0073WOSEQ.txt, created on January 29, 2008 which is 1.25Kb in size. The information in the electronic format of the sequence listing is incorporated herein by reference in its entirety.

### Field of the Invention

The present invention provides methods and compositions for modulating protein expression.

### Background of the Invention

Antisense compounds have been used to modulate target nucleic acids. Antisense compounds comprising a variety of modifications and motifs have been reported. In certain instances, such compounds are useful as research tools and as therapeutic agents. Certain double-stranded RNA compounds (siRNAs) are known to inhibit protein expression in cells. Such double-stranded RNA compounds function, at least in part, through the RNA-inducing silencing complex (RISC).

### Summary of the Invention

In certain embodiments, the present invention provides single-stranded oligomeric compounds that inhibit protein expression. In certain embodiments, oligomeric compounds of the present invention function, at least in part, through the RISC pathway. Single-stranded oligomeric compounds have advantages over double-stranded compounds. For example, such single-stranded compounds may be manufactured at reduced cost and/or may result in fewer and/or less severe side-effects when administered to an animal as a therapeutic agent.

In certain embodiments, the present invention provides an oligomeric compound comprising a single-stranded oligonucleotide consisting of 17-24 linked nucleosides wherein a plurality of the nucleosides are a modified nucleosides, each of the 6-13 3'-most internucleoside linkages is a phosphorothioate linkage and each of the other internucleoside linkages is a phosphodiester linkage. In certain such embodiments, each nucleoside of the oligonucleotide is a 2'-F nucleoside. In certain embodiments, the invention provides pharmaceutical compositions comprising such oligomeric compounds.

In certain embodiments, the invention provides methods of modulating a target protein in a cell by contacting the cell with such an oligomeric compound. In certain such embodiments, the cell is in an animal. In certain embodiments, the invention provides single-stranded oligomeric compounds that activate the RISC pathway. In certain embodiments the invention provides oligomeric compounds that target an mRNA. In certain embodiments, the invention provides oligomeric compounds that function as microRNA compounds (i.e., are microRNA mimetics). In certain embodiments such compounds modulate expression of several target proteins.

### Detailed description of the Invention

It is to be understood that both the foregoing general description and the following detailed description are exemplary and explanatory only and are not restrictive of the invention, as claimed. Herein, the use of the singular includes the plural unless specifically stated otherwise. As used herein, the use of "or" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

The section headings used herein are for organizational purposes only and are not to be construed as limiting the subject matter described. All documents, or portions of documents, cited in this application, including, but not limited to, patents, patent applications, articles, books, and treatises, are hereby expressly incorporated by reference in their entirety for any purpose.

### Definitions

Unless specific definitions are provided, the nomenclature utilized in connection with, and the procedures and techniques of, analytical chemistry, synthetic organic chemistry, and medicinal and pharmaceutical chemistry described herein are those well known and commonly used in the art. Standard techniques may be used for chemical synthesis, and chemical analysis. Certain such techniques and procedures may be found for example in "Carbohydrate Modifications in Antisense Research" Edited by Sangvi and Cook, American Chemical Society , Washington D.C., 1994; "Remington's Pharmaceutical Sciences," Mack Publishing Co., Easton, Pa., 18th edition, 1990; and "Antisense Drug Technology, Principles, Strategies, and Applications" Edited by Stanley T. Crooke, CRC Press, Boca Raton, Florida; and Sambrook et al., "Molecular Cloning, A laboratory Manual," 2nd Edition, Cold Spring Harbor Laboratory Press, 1989, which are hereby incorporated by reference for any purpose. Where permitted, all patents, applications, published applications and other publications and other data referred to throughout in the disclosure herein are incorporated by reference in their entirety.

Unless otherwise indicated, the following terms have the following meanings:

As used herein, the term "nucleoside" means a compound comprising a nucleobase and a sugar. Nucleosides include, but are not limited to, naturally occurring nucleosides, abasic nucleosides, modified nucleosides, and nucleosides having mimetic bases and/or sugar groups.

As used herein, the term "nucleotide" refers to a compound comprising a nucleobase and a sugar having a phosphate group covalently linked to the sugar. Nucleotides may be modified with any of a variety of substituents.

As used herein, the term "linked nucleosides" includes nucleosides linked through a phosphate group (i.e., linked nucleotides) and those linked without phosphate groups.

As used herein, the term "nucleobase" refers to the base portion of a nucleoside or nucleotide. A nucleobase may comprise any atom or group of atoms capable of hydrogen bonding to a base of another nucleic acid.

As used herein, the term "heterocyclic base moiety" refers to a nucleobase comprising a heterocycle.

As used herein, the term "oligomeric compound" refers to a polymeric structure comprising two or more sub-structures and capable of hybridizing to a region of a nucleic acid molecule. In certain embodiments, oligomeric compounds are oligonucleosides. In certain embodiments, oligomeric compounds are oligonucleotides. In certain embodiments, oligomeric compounds are antisense compounds. In certain embodiments, oligomeric compounds are antidote compounds. In certain embodiments, oligomeric compounds comprise conjugate groups.

As used herein, the term "oligonucleotide" refers to an oligomeric compound comprising a plurality of linked nucleosides. In certain embodiments, one or more nucleotides of an oligonucleotide is modified. In certain embodiments, an oligonucleotide comprises ribonucleic acid (RNA) or deoxyribonucleic acid (DNA). In certain embodiments, oligonucleotides are composed of naturally- and/or non-naturally-occurring nucleobases, sugars and covalent internucleoside linkages, and may further include non-nucleic acid conjugates.

As used herein "internucleoside linkage" refers to a covalent linkage between adjacent nucleosides.

As used herein "naturally occurring internucleoside linkage" refers to a 3' to 5' phosphodiester linkage.

As used herein, the term "antisense compound" refers to an oligomeric compound that is at least partially complementary to a target nucleic acid molecule to which it hybridizes. In certain embodiments, an antisense compound modulates (increases or decreases) expression or amount of a target nucleic acid. In certain embodiments, an antisense compound alters splicing of a target pre-mRNA resulting in a different splice variant. Antisense compounds include, but are not limited to, compounds that are oligonucleotides, oligonucleosides, oligonucleotide analogs, oligonucleotide mimetics, and chimeric combinations of these. Consequently, while all antisense compounds are oligomeric compounds, not all oligomeric compounds are antisense compounds.

As used herein, the term "antisense oligonucleotide" refers to an antisense compound that is an oligonucleotide.

As used herein, the term "antisense activity" refers to any detectable and/or measurable activity attributable to the hybridization of an antisense compound to its target nucleic acid. In certain embodiments, such activity may be an increase or decrease in an amount of a nucleic acid or protein. In certain embodiments, such activity may be a change in the ratio of splice variants of a nucleic acid or protein. Detection and/or measuring of antisense activity may be direct or indirect. For example, in certain embodiments, antisense activity is assessed by detecting and or measuring the amount of target protein or the relative amounts of splice variants of a target protein. In certain embodiments, antisense activity is assessed by detecting and/or measuring the amount of target nucleic acids and/or cleaved target nucleic acids and/or alternatively spliced target nucleic acids.

As used herein the term "detecting antisense activity" or "measuring antisense activity" means that a test for detecting or measuring antisense activity is performed on a particular sample and compared to that of a control sample. Such detection and/or measuring may include values of zero. Thus, if a test for detection of antisense activity results in a finding of no antisense activity (antisense activity of zero), the step of "detecting antisense activity" has nevertheless been performed.

As used herein the term "control sample" refers to a sample that has not been contacted with an antisense compound.

As used herein, the term "oligomeric compound motif" refers to the pattern of unmodified and modified nucleosides or nucleotides or linkages in an oligomeric compound.

As used herein, the term "target protein" refers to a protein, the modulation of which is desired.

As used herein, the term "target gene" refers to a gene encoding a target protein.

As used herein, the term "target nucleic acid" refers to any nucleic acid molecule the expression or activity of which is capable of being modulated by an antisense compound. Target nucleic acids include, but are not limited to, RNA (including, but not limited to pre-mRNA and mRNA or portions thereof) transcribed from DNA encoding a target protein, and also cDNA derived from such RNA, and miRNA. For example, the target nucleic acid can be a cellular gene (or mRNA transcribed from the gene) whose expression is associated with a particular disorder or disease state, or a nucleic acid molecule from an infectious agent.

As used herein, the term "targeting" or "targeted to" refers to the association of an antisense compound to a particular target nucleic acid molecule or a particular region of nucleotides within a target nucleic acid molecule.

As used herein, the term "nucleobase complementarity" refers to a nucleobase that is capable of base pairing with another nucleobase. For example, in DNA, adenine (A) is complementary to thymine (T). For example, in RNA, adenine (A) is complementary to uracil (U). In certain embodiments, complementary nucleobase refers to a nucleobase of an antisense compound that is capable of base pairing with a nucleobase of its target nucleic acid. For example, if a nucleobase at a certain position of an antisense compound is capable of hydrogen bonding with a nucleobase at a certain position of a target nucleic acid, then the position of hydrogen bonding between the oligonucleotide and the target nucleic acid is considered to be complementary at that nucleobase pair.

As used herein, the term "non-complementary nucleobase" refers to a pair of nucleobases that do not form hydrogen bonds with one another or otherwise support hybridization.

As used herein, the term "complementary" refers to the capacity of an oligomeric compound to hybridize to another oligomeric compound or nucleic acid through nucleobase complementarity. In certain embodiments, an antisense compound and its target are complementary to each other when a sufficient number of corresponding positions in each molecule are occupied by nucleobases that can bond with each other to allow stable association between the antisense compound and the target. One skilled in the art recognizes that the inclusion of mismatches is possible without eliminating the ability of the oligomeric compounds to remain in association. Therefore, described herein are antisense compounds that may comprise up to about 20% nucleotides that are mismatched (i.e., are not nucleobase complementary to the corresponding nucleotides of the target). Preferably the antisense compounds contain no more than about 15%, more preferably not more than about 10%, most preferably not more than 5% or no mismatches. The remaining nucleotides are nucleobase complementary or otherwise do not disrupt hybridization (e.g., universal bases). One of ordinary skill in the art would recognize the compounds provided herein are at least 80%, at least 85%, at least 90%, at least 95%, at least 96%, at least 97%, at least 98%, at least 99% or 100% complementary to a target nucleic acid.

As used herein, "hybridization" means the pairing of complementary oligomeric compounds (e.g., an antisense compound and its target nucleic acid or an antidote to its antisense compound). While not limited to a particular mechanism, the most common mechanism of pairing involves hydrogen bonding, which may be Watson-Crick, Hoogsteen or reversed Hoogsteen hydrogen bonding, between complementary nucleoside or nucleotide bases (nucleobases). For example, the natural base adenine is nucleobase complementary to the natural nucleobases thymidine and uracil which pair through the formation of hydrogen bonds. The natural base guanine is nucleobase complementary to the natural bases cytosine and 5-methyl cytosine. Hybridization can occur under varying circumstances.

As used herein, the term "specifically hybridizes" refers to the ability of an oligomeric compound to hybridize to one nucleic acid site with greater affinity than it hybridizes to another nucleic acid site. In certain embodiments, an antisense oligonucleotide specifically hybridizes to more than one target site.

As used herein, "designing" or "designed to" refer to the process of designing an oligomeric compound that specifically hybridizes with a selected nucleic acid molecule.

As used herein, the term "modulation" refers to a perturbation of amount or quality of a function or activity when compared to the function or activity prior to modulation. For example, modulation includes the change, either an increase (stimulation or induction) or a decrease (inhibition or reduction) in gene expression.

As used herein, the term "expression" refers to all the functions and steps by which a gene's coded information is converted into structures present and operating in a cell. Such structures include, but are not limited to the products of transcription and translation.

As used herein, the term "2'-modified" or "2'-substituted" means a nucleoside comprising a sugar comprising a substituent at the 2' position other than H or OH.

As used herein, the term "2'-F nucleoside" refers to a nucleoside comprising a fluorine at the 2' position of the sugar moiety.

As used herein, the term "prodrug" refers to a therapeutic agent that is prepared in an inactive form that is converted to an active form (i.e., drug) within the body or cells thereof by the action of endogenous enzymes or other chemicals and/or conditions.

As used herein, the term "pharmaceutically acceptable salts" refers to salts of active compounds that retain the desired biological activity of the active compound and do not impart undesired toxicological effects thereto.

As used herein, the term "cap structure" or "terminal cap moiety" refers to chemical modifications, which have been incorporated at either terminus of an antisense compound.

As used herein, the term "prevention" refers to delaying or forestalling the onset or development of a condition or disease for a period of time from hours to days, preferably weeks to months.

As used herein, the term "amelioration" refers to a lessening of at least one activity or one indicator of the severity of a condition or disease. The severity of indicators may be determined by subjective or objective measures which are known to those skilled in the art.

As used herein, the term "treatment" refers to administering a composition of the invention to effect an alteration or improvement of the disease or condition. Prevention, amelioration, and/or treatment may require administration of multiple doses at regular intervals, or prior to onset of the disease or condition to alter the course of the disease or condition. Moreover, a single agent may be used in a single individual for each prevention, amelioration, and treatment of a condition or disease sequentially, or concurrently.

As used herein, the term "pharmaceutical agent" refers to a substance provides a therapeutic benefit when administered to a subject.

As used herein, the term "therapeutically effective amount" refers to an amount of a pharmaceutical agent that provides a therapeutic benefit to an animal.

As used herein, "administering" means providing a pharmaceutical agent to an animal, and includes, but is not limited to administering by a medical professional and self-administering.

As used herein, the term "pharmaceutical composition" refers to a mixture of substances suitable for administering to an individual. For example, a pharmaceutical composition may comprise an antisense oligonucleotide and a sterile aqueous solution.

As used herein, the term "animal" refers to a human or non-human animal, including, but not limited to, mice, rats, rabbits, dogs, cats, pigs, and non-human primates, including, but not limited to, monkeys and chimpanzees.

As used herein, the term "parenteral administration," refers to administration through injection or infusion. Parenteral administration includes, but is not limited to, subcutaneous administration, intravenous administration, or intramuscular administration.

As used herein, the term "subcutaneous administration" refers to administration just below the skin. "Intravenous administration" means administration into a vein.

As used herein, the term "dose" refers to a specified quantity of a pharmaceutical agent provided in a single administration. In certain embodiments, a dose may be administered in two or more boluses, tablets, or injections. For example, in certain embodiments, where subcutaneous administration is desired, the desired dose requires a volume not easily accommodated by a single injection. In such embodiments, two or more injections may be used to achieve the desired dose. In certain embodiments, a dose may be administered in two or more injections to minimize injection site reaction in an individual.

As used herein, the term "dosage unit" refers to a form in which a pharmaceutical agent is provided. In certain embodiments, a dosage unit is a vial comprising lyophilized antisense oligonucleotide. In certain embodiments, a dosage unit is a vial comprising reconstituted antisense oligonucleotide.

As used herein, the term "active pharmaceutical ingredient" refers to the substance in a pharmaceutical composition that provides a desired effect.

### Certain compounds

In certain embodiments, the present invention provides oligomeric compounds. In certain such embodiments, oligomeric compounds have antisense activity. Oligomeric compounds of the present invention may comprise any of a number of oligomeric compound motifs. In certain embodiments, oligomeric compounds comprise oligonucleotides, which constitute linked nucleosides, and optionally comprise one or more conjugate and/or capping group.

### Certain oligomeric compounds

In certain embodiments, nucleosides, including, modified nucleosides, are linked using modified and/or unmodified linkages to form oligomeric compounds. Such oligomeric compounds may be described by motif. In certain embodiments, oligomeric compounds are described by overall length, nucleoside motif, and linkage motif. For example, in certain embodiments, oligomeric compounds are 17-24 nucleosides in length, have a nucleoside motif of full 2'-F nucleosides, and have a linkage motif of 7 phosphorothioates at the 3'end.

### Certain oligomeric compound lengths

In certain embodiments, the present invention provides oligomeric compounds, including antisense oligomeric compounds, of any of a variety of ranges of lengths. In certain embodiments, the invention provides oligomeric compounds consisting of X-Y linked oligonucleosides, where X and Y are each independently selected from 11, 12, 13, 14, 15, 16, 17, 18, 19, 20, 21, 22, 23, 24, 25, 26, 27, 28, 29, and 30; provided that X<Y. For example, in certain embodiments, the invention provides oligomeric compounds comprising: 11-12, 11-13, 11-14, 11-15, 11-16, 11-17, 11-18, 11-19, 11-20, 11-21, 11-22, 11-23, 11-24, 11-25, 11-26, 11-27, 12-13, 12-14, 12-15, 12-16, 12-17, 12-18, 12-19, 12-20, 12-21, 12-22, 12-23, 12-24, 12-25, 12-26, 12-27, 13-14, 13-15, 13-16, 13-17, 13-18, 13-19, 13-20, 13-21, 13-22, 13-23, 13-24, 13-25, 13-26, 13-27, 14-15, 14-16, 14-17, 14-18, 14-19, 14-20, 14-21, 14-22, 14-23, 14-24, 14-25, 14-26, 14-27, 15-16, 15-17, 15-18, 15-19, 15-20, 15-21, 15-22, 15-23, 15-24, 15-25, 15-26, 15-27, 16-17, 16-18, 16-19, 16-20, 16-21, 16-22, 16-23, 16-24, 16-25, 16-26, 16-27, 17-18, 17-19, 17-20, 17-21, 17-22, 17-23, 17-24, 17-25, 17-26, 17-27, 18-19, 18-20, 18-21, 18-22, 18-23, 18-24, 18-25, 18-26, 18-27, 19-20, 19-21, 19-22, 19-23, 19-24, 19-25, 19-26, 20-21, 20-22, 20-23, 20-24, 20-25, 20-26, 20-27, 21-22, 21-23, 21-24, 21-25, 21-26, 21-27, 22-23, 22-24, 22-25, 22-26, 22-27, 23-24, 23-25, 23-26, 23-27, 24-25, 24-26, 24-27, 25-26, 25-27, or 26-27 linked nucleosides.

### Certain linkage motifs

In certain embodiments, oligomeric compounds of the present invention comprise 5 modified linkages at the 3' end, and the remaining linkages are natural phosphodiester linkages. In certain embodiments, oligomeric compounds of the present invention comprise 6 modified linkages at the 3' end, and the remaining linkages are natural phosphodiester linkages. In certain embodiments, oligomeric compounds of the present invention comprise 7 modified linkages at the 3' end, and the remaining linkages are natural phosphodiester linkages. In certain embodiments, oligomeric compounds of the present invention comprise up to 8 modified linkages at the 3' end, and the remaining linkages are natural phosphodiester linkages. In certain embodiments, oligomeric compounds of the present invention comprise up to 9 modified linkages at the 3' end, and the remaining linkages are natural phosphodiester linkages. In certain embodiments, oligomeric compounds of the present invention comprise up to 10 modified linkages at the 3' end, and the remaining linkages are natural phosphodiester linkages. In certain embodiments, oligomeric compounds of the present invention comprise up to 11 modified linkages at the 3' end, and the remaining linkages are natural phosphodiester linkages. In certain embodiments, oligomeric compounds of the present invention comprise up to 12 modified linkages at the 3' end, and the remaining linkages are natural phosphodiester linkages. In certain embodiments, oligomeric compounds of the present invention comprise up to 13 modified linkages at the 3' end, and the remaining linkages are natural phosphodiester linkages. In any of those embodiments comprising 5, 6, 7, 8, 9, 10, 11, 12, or 13 modified linkages at the 3' end, each of those modified linkages may be a phosphorothioate linkage. Thus, in certain embodiments in which an oligomeric compound comprises between 17 and 24 linked nucleosides, such oligomeric compounds have a linkage motif selected from:
5'-(N-o-)ₓ(N-s-)_{y}N-3'
where N is any nucleoside;
o is a phosphodiester linkage
s is a phosphorothioate linkage
x is 4-19; and y is 5-13;
where the sum of x and y is between 16 and 23.
For example, certain such oligomeric compound have a linkage motif selected from: NoNoNoNoNoNoNoNoNoNoNoNoNsNsNsNsNsNsN (19-mer with 6 3'-terminal P=S) (SEQ ID NO: 1) NoNoNoNoNoNoNoNoNoNoNoNsNsNsNsNsNsNsN (19-mer with 7 3'-terminal P=S) (SEQ ID NO: 1) NoNoNoNoNoNoNoNoNoNoNsNsNsNsNsNsNsNsN (19-mer with 8 3'-terminal P=S) (SEQ ID NO: 1) NoNoNoNoNoNoNoNoNoNsNsNsNsNsNsNsNsNsN (19-mer with 9 3'-terminal P=S) (SEQ ID NO: 1) NoNoNoNoNoNoNoNoNoNoNoNoNoNsNsNsNsNsNsN (20-mer with 6 3'-terminal P=S) (SEQ ID NO: 2) NoNoNoNoNoNoNoNoNoNoNoNoNsNsNsNsNsNsNsN (20-mer with 7 3'-terminal P=S) (SEQ ID NO: 2) NoNoNoNoNoNoNoNoNoNoNoNsNsNsNsNsNsNsNsN (20-mer with 8 3'-terminal P=S) (SEQ ID NO: 2) NoNoNoNoNoNoNoNoNoNoNsNsNsNsNsNsNsNsNsN (20-mer with 9 3'-terminal P=S) (SEQ ID NO: 2) One of ordinary skill in the art will readily appreciate that longer or shorter oligomeric compounds may be designed. Likewise, one skilled in the art will readily appreciate that additional linkages may be phosphorothioate. In certain embodiments, each nucleoside in the above linkage motifs is a 2'F-modified nucleoside.

### Conjugated Oligomeric Compounds

In certain embodiments, oligomeric compounds comprise one or more moieties, capping groups and/or conjugates. In certain embodiments, oligomeric compounds of the invention comprise the linkage of one or more moieties or conjugates which enhance the activity, cellular distribution or cellular uptake of the resulting oligomeric compounds. In certain such embodiments such modified oligomeric compounds are prepared by covalently attaching conjugate groups to functional groups such as hydroxyl or amino groups. Conjugate groups of the invention include intercalators, reporter molecules, polyamines, polyamides, polyethylene glycols, polyethers, groups that enhance the pharmacodynamic properties of oligomers, and groups that enhance the pharmacokinetic properties of oligomers. Typical conjugates groups include cholesterols, carbohydrates, lipids, phospholipids, biotin, phenazine, folate, phenanthridine, anthraquinone, acridine, fluoresceins, rhodamines, coumarins, and dyes. Groups that enhance the pharmacodynamic properties, in the context of this invention, include groups that improve oligomeric compound uptake, enhance oligomeric compound resistance to degradation, and/or strengthen hybridization with RNA. Groups that enhance the pharmacokinetic properties, in the context of this invention, include groups that improve oligomeric compound uptake, distribution, metabolism or excretion. Representative conjugate groups are disclosed in International Patent Application PCT/US92/09196, filed October 23, 1992 the entire disclosure of which is incorporated herein by reference. Conjugate moieties include but are not limited to lipid moieties such as a cholesterol moiety and a variety of others known in the art.

Furthermore, the oligomeric compounds of the invention can have one or more moieties bound or conjugated, which facilitates the active or passive transport, localization, or compartmentalization of the oligomeric compound. Cellular localization includes, but is not limited to, localization to within the nucleus, the nucleolus, or the cytoplasm. Compartmentalization includes, but is not limited to, any directed movement of the oligonucleotides of the invention to a cellular compartment including the nucleus, nucleolus, mitochondrion, or imbedding into a cellular membrane. Furthermore, the oligomeric compounds of the invention comprise one or more conjugate moieties which facilitate posttranscriptional modification.

Certain conjugate groups amenable to the present invention include lipid moieties such as a cholesterol moiety (Letsinger et al., Proc. Natl. Acad. Sci. USA, 1989, 86, 6553); cholic acid (Manoharan et al., Bioorg. Med. Chem. Lett., 1994, 4, 1053); a thioether, e.g., hexyl-S-tritylthiol (Manoharan et al., Ann. N.Y. Acad. Sci., 1992, 660, 306; Manoharan et al., Bioorg. Med. Chem. Let., 1993, 3, 2765); a thiocholesterol (Oberhauser et al., Nucl. Acids Res., 1992, 20, 533); an aliphatic chain, e.g., dodecandiol or undecyl residues (Saison-Behmoaras et al., EMBO J., 1991, 10, 111; Kabanov et al., FEBS Lett., 1990, 259, 327; Svinarchuk et al., Biochimie, 1993, 75, 49); a phospholipid, e.g., di-hexadecyl-rac-glycerol or triethyl-ammonium-1,2-di-O-hexadecyl-rac-glycero-3-H-phosphonate (Manoharan et al., Tetrahedron Lett., 1995, 36, 3651; Shea et al., Nucl. Acids Res., 1990, 18, 3777); a polyamine or a polyethylene glycol chain (Manoharan et al., Nucleosides & Nucleotides, 1995, 14, 969); adamantane acetic acid (Manoharan et al., Tetrahedron Lett., 1995, 36, 3651); a palmityl moiety (Mishra et al., Biochim. Biophys. Acta, 1995, 1264, 229); or an octadecylamine or hexylamino-carbonyl-oxycholesterol moiety (Crooke et al., J. Pharmacol. Exp. Ther., 1996,277,923).

Conjugate groups can be attached to various positions of an oligomeric compound directly or via an optional linking group. The term linking group is intended to include all groups amenable to attachment of a conjugate group to an oligomeric compound. Linking groups are bivalent groups useful for attachment of chemical functional groups, conjugate groups, reporter groups and other groups to selective sites in a parent compound such as for example an oligomeric compound. In general a bifunctional linking moiety comprises a hydrocarbyl moiety having two functional groups. One of the functional groups is selected to bind to a parent molecule or compound of interest and the other is selected to bind essentially any selected group such as chemical functional group or a conjugate group. In some embodiments, the linker comprises a chain structure or an oligomeric compound of repeating units such as ethylene glyol or amino acid units. Examples of functional groups that are routinely used in bifunctional linking moieties include, but are not limited to, electrophiles for reacting with nucleophilic groups and nucleophiles for reacting with electrophilic groups. In some embodiments, bifunctional linking moieties include amino, hydroxyl, carboxylic acid, thiol, unsaturations (e.g., double or triple bonds), and the like. Some nonlimiting examples of bifunctional linking moieties include 8-amino-3,6-dioxaoctanoic acid (ADO), succinimidyl 4-(N-maleimidomethyl) cyclohexane-1-carboxylate (SMCC) and 6-aminohexanoic acid (AHEX or AHA). Other linking groups include, but are not limited to, substituted C1-C10 alkyl, substituted or unsubstituted C2-C10 alkenyl or substituted or unsubstituted C2-C10 alkynyl, wherein a nonlimiting list of preferred substituent groups includes hydroxyl, amino, alkoxy, carboxy, benzyl, phenyl, nitro, thiol, thioalkoxy, halogen, alkyl, aryl, alkenyl and alkynyl. Further representative linking groups are disclosed for example in WO 94/01550 and WO 94/01550.

One representative formula for a linked conjugate is shown below for illustration and is not limiting:

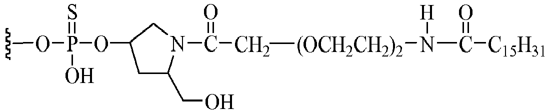

The formula includes a C₁₆ lipophilic conjugate attached via a pyrrolidinyl linker to a phosphororthioate group. The phosphorothioate group can be attached directly to the 3'-end of an oligomeric compound or can be attached to a 3'-terminal non hybridizing nucleoside.

Oligomeric compounds used in the compositions of the present invention can also be modified to have one or more stabilizing groups that are generally attached to one or both termini of oligomeric compounds to enhance properties such as for example nuclease stability. Included in stabilizing groups are cap structures. By "cap structure or terminal cap moiety" is meant chemical modifications, which have been incorporated at either terminus of oligonucleotides (see for example Wincott et al., WO 97/26270, incorporated by reference herein). These terminal modifications can protect the oligomeric compounds having terminal nucleic acid molecules from exonuclease degradation, and can help in delivery and/or localization within a cell. The cap can be present at the 5'-terminus (5'-cap) or at the 3'-terminus (3'-cap) or can be present on both termini. In non-limiting examples, the 5'-cap includes inverted abasic residue (moiety), 4',5'-methylene nucleotide; 1-(beta-D-erythrofuranosyl) nucleotide, 4'-thio nucleotide, carbocyclic nucleotide; 1,5-anhydrohexitol nucleotide; L-nucleotides; alpha-nucleotides; modified base nucleotide; phosphorodithioate linkage; threo-pentofuranosyl nucleotide; acyclic 3',4'-seco nucleotide; acyclic 3,4-dihydroxybutyl nucleotide; acyclic 3,5-dihydroxypentyl riucleotide, 3'-3'-inverted nucleotide moiety; 3'-3'-inverted abasic moiety; 3'-2'-inverted nucleotide moiety; 3'-2'-inverted abasic moiety; 1,4-butanediol phosphate; 3'-phosphoramidate; hexylphosphate; aminohexyl phosphate; 3'-phosphate; 3'-phosphorothioate; phosphorodithioate; or bridging or non-bridging methylphosphonate moiety (see Wincott et al., International PCT publication No. WO 97/26270, incorporated by reference herein).

In certain embodiments, oligomeric compounds comprise a 3'-cap structure selected from 4',5'-methylene nucleotide; 1-(beta-D-erythrofuranosyl) nucleotide; 4'-thio nucleotide, carbocyclic nucleotide; 5'-amino-alkyl phosphate; 1,3-diamino-2-propyl phosphate, 3-aminopropyl phosphate; 6-aminohexyl phosphate; 1,2-aminododecyl phosphate; hydroxypropyl phosphate; 1,5-anhydrohexitol nucleotide; L-nucleotide; alpha-nucleotide; modified base nucleotide; phosphorodithioate; threo-pentofuranosyl nucleotide; acyclic 3',4'-seco nucleotide; 3,4-dihydroxybutyl nucleotide; 3,5-dihydroxypentyl nucleotide, 5'-5'-inverted nucleotide moiety; 5'-5'-inverted abasic moiety; 5'-phosphoramidate; 5'-phosphorothioate; 1,4-butanediol phosphate; 5'-amino; bridging and/or non-bridging 5'-phosphoramidate, phosphorothioate and/or phosphorodithioate, bridging or non bridging methylphosphonate and 5'-mercapto moieties (for more details see Beaucage and Tyer, 1993, Tetrahedron 49, 1925; incorporated by reference herein).

Further 3' and 5'-stabilizing groups that can be used to cap one or both ends of an oligomeric compound to impart nuclease stability include those disclosed in WO 03/004602 published on January 16, 2003.

Non-limiting examples of oligomeric compounds of the present invention are provided below: P-N_{fo}N_{fo}N_{fo}N_{fo}N_{fo}N_{fo}N_{fo}N_{fo}N_{fo}N_{fo}N_{fo}N_{fo}N_{fs}N_{fs}N_{fs}N_{fs}N_{fs}N_{fs}N_{f} (SEQ ID NO: 1) P-N_{fo}N_{fo}N_{fo}N_{fo}N_{fo}N_{fo}N_{fo}N_{fo}N_{fo}N_{fo}N_{fo}N_{fs}N_{fs}N_{fs}N_{fs}N_{fs}N_{fs}N_{fs}N_{f} (SEQ ID NO: 1) P-N_{fo}N_{fo}N_{fo}N_{fo}N_{fo}N_{fo}N_{fo}N_{fo}N_{fo}N_{fo}N_{fs}N_{fs}N_{fs}N_{fs}N_{fs}N_{fs}N_{fs}N_{fs}N_{f} (SEQ ID NO: 1) P-N_{fo}N_{fo}N_{fo}N_{fo}N_{fo}N_{fo}N_{fo}N_{fo}N_{fo}N_{fo}N_{fo}N_{fo}N_{fs}N_{fs}N_{fs}N_{fs}N_{fs}N_{fs}N_{f-}C16 (SEQ ID NO: 1) P-N_{fo}N_{fo}N_{fo}N_{fo}N_{fo}N_{fo}N_{fo}N_{fo}N_{fo}N_{fo}N_{fo}N_{fs}N_{fs}N_{fs}N_{fs}N_{fs}N_{fs}N_{fs}N_{f}-C16 (SEQ ID NO: 1) P-N_{fo}N_{fo}N_{fo}N_{fo}N_{fo}N_{fo}N_{fo}N_{fo}N_{fo}N_{fo}N_{fs}N_{fs}N_{fs}N_{fs}N_{fs}N_{fs}N_{fs}N_{fs}N_{f}-C16 (SEQ ID NO: 1) P-N_{fo}N_{fo}N_{fo}N_{fo}N_{fo}N_{fo}N_{fo}N_{fo}N_{fo}N_{fo}N_{fo}N_{fo}N_{fo}N_{fs}N_{fs}N_{fs}N_{fs}N_{fs}N_{fs}N_{f} (SEQ ID NO: 2) P-N_{fo}N_{fo}N_{fo}N_{fo}N_{fo}N_{fo}N_{fo}N_{fo}N_{fo}N_{fo}N_{fo}N_{fo}N_{fs}N_{fs}N_{fs}N_{fs}N_{fs}N_{fs}N_{fs}N_{f} (SEQ ID NO: 2) P-N_{fo}N_{fo}N_{fo}N_{fo}N_{fo}N_{fo}N_{fo}N_{fo}N_{fo}N_{fo}N_{fo}N_{fs}N_{fs}N_{fs}N_{fs}N_{fs}N_{fs}N_{fs}N_{fs}N_{f} (SEQ ID NO: 2) P-N_{fo}N_{fo}N_{fo}N_{fo}N_{fo}N_{fo}N_{fo}N_{fo}N_{fo}N_{fo}N_{fo}N_{fo}N_{fo}N_{fs}N_{fs}N_{fs}N_{fs}N_{fs}N_{fs}N_{f}-C16 (SEQ ID NO: 2) P-N_{fo}N_{fo}N_{fo}N_{fo}N_{fo}N_{fo}N_{fo}N_{fo}N_{fo}N_{fo}N_{fo}N_{fo}N_{fs}N_{fs}N_{fs}N_{fs}N_{fs}N_{fs}N_{fs}N_{f-}C16 (SEQ ID NO: 2) P-N_{fo}N_{fo}N_{fo}N_{fo}N_{fo}N_{fo}N_{fo}N_{fo}N_{fo}N_{fo}N_{fo}N_{fs}N_{fs}N_{fs}N_{fs}N_{fs}N_{fs}N_{fs}N_{fs}N_{f}-C16 (SEQ ID NO: 2) P-N_{fo}N_{fo}N_{fo}N_{fo}N_{fo}N_{fo}N_{fo}N_{fo}N_{fo}N_{fo}N_{fo}N_{fo}N_{fo}N_{fo}N_{fs}N_{fs}N_{fs}N_{fs}N_{fs}NfₛN_{f} (SEQ ID NO: 3) P-N_{fo}N_{fo}N_{fo}N_{fo}N_{fo}N_{fo}N_{fo}N_{fo}N_{fo}N_{fo}N_{fo}N_{fo}N_{fo}N_{fs}N_{fs}N_{fs}N_{fs}N_{fs}N_{fs}N_{fs}N_{f} (SEQ ID NO: 3) P-N_{fo}N_{fo}N_{fo}N_{fo}N_{fo}N_{fo}N_{fo}N_{fo}N_{fo}N_{fo}N_{fo}N_{fo}N_{fs}N_{fs}N_{fs}N_{fs}N_{fs}N_{fs}N_{fs}N_{fs}N_{f} (SEQ ID NO: 3) P-N_{fo}N_{fo}N_{fo}N_{fo}N_{fo}N_{fo}N_{fo}N_{fo}N_{fo}N_{fo}N_{fo}N_{fo}N_{fo}N_{fo}N_{fs}N_{fs}N_{fs}N_{fs}N_{fs}N_{fs}N_{f-}C16 (SEQ ID NO: 3) P-N_{fo}N_{fo}N_{fo}N_{fo}N_{fo}N_{fo}N_{fo}N_{fo}N_{fo}N_{fo}N_{fo}N_{fo}N_{fo}N_{fs}N_{fs}N_{fs}N_{fs}N_{fs}N_{fs}N_{fs}N_{f}-C16 (SEQ ID NO: 3) P-N_{fo}N_{fo}N_{fo}N_{fo}N_{fo}N_{fo}N_{fo}N_{fo}N_{fo}N_{fo}N_{fo}N_{fo}N_{fs}N_{fs}N_{fs}N_{fs}N_{fs}N_{fs}N_{fs}N_{fs}N_{f}-C16 (SEQ ID NO: 3) where P is a phosphate; C16 is the C₁₆ lipophilic conjugate described above; N_{f} is a 2'-F nucleoside; o is phosphodiester linkage; and s is phosphorothioate linkage. In certain embodiments, the 1-3 3'terminal nucleosides of an oligomeric compound are not complementary to a target nucleic acid. In certain such embodiments, the 3'terminal nucleosides are 2'-U_{f}, regardless of the corresponding base of the target nucleic acid.

### Synthesis, Purification and Analysis

Oligomerization of modified and unmodified nucleosides and nucleotides can be routinely performed according to literature procedures for DNA (Protocols for Oligonucleotides and Analogs, Ed. Agrawal (1993), Humana Press) and/or RNA (Scaringe, Methods (2001), 23, 206-217. Gait et al., Applications of Chemically synthesized RNA in RNA: Protein Interactions, Ed. Smith (1998), 1-36. Gallo et al., Tetrahedron (2001), 57, 5707-5713).

Oligomeric compounds provided herein can be conveniently and routinely made through the well-known technique of solid phase synthesis. Equipment for such synthesis is sold by several vendors including, for example, Applied Biosystems (Foster City, CA). Any other means for such synthesis known in the art may additionally or alternatively be employed. It is well known to use similar techniques to prepare oligonucleotides such as the phosphorothioates and alkylated derivatives. The invention is not limited by the method of antisense compound synthesis.

Methods of purification and analysis of oligomeric compounds are known to those skilled in the art. Analysis methods include capillary electrophoresis (CE) and electrospray-mass spectroscopy. Such synthesis and analysis methods can be performed in multi-well plates. The method of the invention is not limited by the method of oligomeric compound purification.

### Compositions and Methods for Formulating Pharmaceutical Compositions

Oligomeric compounds may be admixed with pharmaceutically acceptable active and/or inert substances for the preparation of pharmaceutical compositions or formulations. Compositions and methods for the formulation of pharmaceutical compositions are dependent upon a number of criteria, including, but not limited to, route of administration, extent of disease, or dose to be administered.

Oligomeric compounds, including antisense compounds and/or antidote compounds, can be utilized in pharmaceutical compositions by combining such oligomeric compounds with a suitable pharmaceutically acceptable diluent or carrier. A pharmaceutically acceptable diluent includes phosphate-buffered saline (PBS). PBS is a diluent suitable for use in compositions to be delivered parenterally. Accordingly, in one embodiment, employed in the methods described herein is a pharmaceutical composition comprising an antisense compound and/or antidote compound and a pharmaceutically acceptable diluent. In certain embodiments, the pharmaceutically acceptable diluent is PBS.

Pharmaceutical compositions comprising oligomeric compounds encompass any pharmaceutically acceptable salts, esters, or salts of such esters. In certain embodiments, pharmaceutical compositions comprising oligomeric compounds comprise one or more oligonucleotide which, upon administration to an animal, including a human, is capable of providing (directly or indirectly) the biologically active metabolite or residue thereof. Accordingly, for example, the disclosure is also drawn to pharmaceutically acceptable salts of antisense compounds, prodrugs, pharmaceutically acceptable salts of such prodrugs, and other bioequivalents. Suitable pharmaceutically acceptable salts include, but are not limited to, sodium and potassium salts.

A prodrug can include the incorporation of additional nucleosides at one or both ends of an oligomeric compound which are cleaved by endogenous nucleases within the body, to form the active oligomeric compound.

### Certain methods

In certain embodiments, oligomeric compounds of the present invention have antisense activity. In certain embodiments, oligomeric compounds modulate expression of a target protein. In certain embodiments, oligomeric compounds modulate the amount of target nucleic acid and/or target protein in a cell. In certain embodiments, a target nucleic acid is a mRNA. In certain embodiments, a target nucleic acid is a non-coding RNA. In certain such embodiments, a target nucleic acid is a microRNA. In certain embodiments, oligomeric compounds are microRNA mimetics.

In certain embodiments, oligomeric compounds hybridize to a target nucleic acid. In certain such embodiments, such binding results in cleavage of the target nucleic acid. In embodiments in which the target nucleic acid is an mRNA, such binding and cleavage typically results in a decrease in the concentration of the protein encoded by such mRNA.

In the context of this invention, "hybridization" means hydrogen bonding, which may be Watson-Crick, Hoogsteen or reversed Hoogsteen hydrogen bonding, between the heterocyclic base moieties of complementary nucleosides. For example, adenine and thymine are complementary nucleobases which pair through the formation of hydrogen bonds. "Complementary," as used herein, refers to the capacity for precise pairing between two nucleotides. For example, if a nucleotide at a certain position of an oligonucleotide is capable of hydrogen bonding with a nucleotide at the same position of a DNA or RNA molecule, then the oligonucleotide and the DNA or RNA are considered to be complementary to each other at that position. The oligonucleotide and the DNA or RNA are complementary to each other when a sufficient number of corresponding positions in each molecule are occupied by nucleotides which can hydrogen bond with each other. Thus, "specifically hybridizable" and "complementary" are terms which are used to indicate a sufficient degree of complementarity or precise pairing such that stable and specific binding occurs between the oligonucleotide and the DNA or RNA target. It is understood in the art that the sequence of an antisense oligomeric compound need not be 100% complementary to that of its target nucleic acid to be specifically hybridizable. An antisense oligomeric compound is specifically hybridizable when binding of the compound to the target DNA or RNA molecule interferes with the normal function of the target DNA or RNA to cause a complete or partial loss of function, and there is a sufficient degree of complementarity to avoid non-specific binding of the antisense oligomeric compound to non-target sequences under conditions in which specific binding is desired, i.e., under physiological conditions in the case of therapeutic treatment, or under conditions in which *in vitro* or *in vivo* assays are performed. Moreover, an oligonucleotide may hybridize over one or more segments such that intervening or adjacent segments are not involved in the hybridization event (e.g., a loop structure, mismatch or hairpin structure).

The oligomeric compounds of the present invention comprise at least 70%, at least 75%, at least 80%, at least 85%, at least 90%, at least 95%, at least 99%, or 100% sequence complementarity to a target region within the target nucleic acid sequence to which they are targeted. For example, an antisense oligomeric compound in which 18 of 20 nucleobases of the antisense oligomeric compound are complementary to a target region, and would therefore specifically hybridize, would represent 90 percent complementarity. In this example, the remaining noncomplementary nucleobases may be clustered or interspersed with complementary nucleobases and need not be contiguous to each other or to complementary nucleobases. As such, an antisense oligomeric compound which is 18 nucleobases in length having 4 (four) noncomplementary nucleobases which are flanked by two regions of complete complementarity with the target nucleic acid would have 77.8% overall complementarity with the target nucleic acid and would thus fall within the scope of the present invention.

Percent complementarity of an antisense oligomeric compound with a region of a target nucleic acid can be determined routinely using BLAST programs (basic local alignment search tools) and PowerBLAST programs known in the art (Altschul et al., J. Mol. Biol., 1990, 215, 403-410; Zhang and Madden, Genome Res., 1997, 7, 649-656). Percent homology, sequence identity or complementarity, can be determined by, for example, the Gap program (Wisconsin Sequence Analysis Package, Version 8 for Unix, Genetics Computer Group, University Research Park, Madison WI), using default settings, which uses the algorithm of Smith and Waterman (Adv. Appl. Math., 1981, 2, 482-489). In some embodiments, homology, sequence identity or complementarity, between the oligomeric compound and the target is about 70%, about 75%, about 80%, about 85%, about 90%, about 92%, about 94%, about 95%, about 96%, about 97%, about 98%, about 99%, or 100%.

In certain embodiments, oligomeric compounds comprise oligonucleotides complementary to a target nucleic acid and further comprise conjugates and/or capping groups. In certain embodiments, those conjugates and/or capping groups are terminal nucleosides. In such embodiments, such terminal nucleosides may or may not hybridize to the target nucleic acid. In such embodiments, such terminal nucleosides are not used when determining percent complementarity or when determining the length of the oligonucleotide. In double-stranded siRNA duplexes, such nucleosides constitute an overhang, because they are typically not present in the complementary strand of the duplex. In a single-stranded oligomeric compound, such nucleosides are simply terminal nucleosides. Typically, such nucleosides are on the 3' end. In certain embodiments, such terminal nucleosides comprise any modification, including those present in the oligonucleotide and those not otherwise present in the oligomeric compound.

In some embodiments, "suitable target segments" may be employed in a screen for additional oligomeric compounds that modulate the expression of a selected protein. "Modulators" are those oligomeric compounds that decrease or increase the expression of a nucleic acid molecule encoding a protein and which comprise at least an 8-nucleobase portion which is complementary to a suitable target segment. The screening method comprises the steps of contacting a suitable target segment of a nucleic acid molecule encoding a protein with one or more candidate modulators, and selecting for one or more candidate modulators which decrease or increase the expression of a nucleic acid molecule encoding a protein. Once it is shown that the candidate modulator or modulators are capable of modulating (e.g. either decreasing or increasing) the expression of a nucleic acid molecule encoding a peptide, the modulator may then be employed in further investigative studies of the function of the peptide, or for use as a research, diagnostic, or therapeutic agent in accordance with the present invention.

The suitable target segments of the present invention may also be combined with their respective complementary antisense oligomeric compounds of the present invention to form stabilized double stranded (duplexed) oligonucleotides. Such double stranded oligonucleotide moieties have been shown in the art to modulate target expression and regulate translation as well as RNA processsing via an antisense mechanism. Moreover, the double stranded moieties may be subject to chemical modifications (Fire et al., Nature, 1998, 391, 806-811; Timmons and Fire, Nature 1998, 395, 854; Timmons et al., Gene, 2001, 263, 103-112; Tabara et al., Science, 1998, 282, 430-431; Montgomery et al., Proc. Natl. Acad. Sci. USA, 1998, 95, 15502-15507; Tuschl et al., Genes Dev., 1999, 13, 3191-3197; Elbashir et al., Nature, 2001, 411, 494-498; Elbashir et al., Genes Dev. 2001, 15, 188-200). For example, such double stranded moieties have been shown to inhibit the target by the classical hybridization of antisense strand of the duplex to the target, thereby triggering enzymatic degradation of the target (Tijsterman et al., Science, 2002, 295, 694-697). The oligomeric compounds of the present invention can also be applied in the areas of drug discovery and target validation. The present invention comprehends the use of the oligomeric compounds and targets identified herein in drug discovery efforts to elucidate relationships that exist between proteins and a disease state, phenotype, or condition. These methods include detecting or modulating a target peptide comprising contacting a sample, tissue, cell, or organism with the oligomeric compounds of the present invention, measuring the nucleic acid or protein level of the target and/or a related phenotypic or chemical endpoint at some time after treatment, and optionally comparing the measured value to a non-treated sample or sample treated with a further oligomeric compound of the invention. These methods can also be performed in parallel or in combination with other experiments to determine the function of unknown genes for the process of target validation or to determine the validity of a particular gene product as a target for treatment or prevention of a particular disease, condition, or phenotype.

Effect of nucleoside modifications on RNAi activity can be evaluated according to existing literature (Elbashir et al., Nature, 2001,411,494-498; Nishikura et al., Cell, 2001, 107, 415-416; and Bass et al., Cell, 2000, 101, 235-238.)

The oligomeric compounds of the present invention can be utilized for diagnostics, therapeutics, prophylaxis and as research reagents and kits. Furthermore, antisense oligonucleotides, which are able to inhibit gene expression with exquisite specificity, are often used by those of ordinary skill to elucidate the function of particular genes or to distinguish between functions of various members of a biological pathway. For use in kits and diagnostics, the oligomeric compounds of the present invention, either alone or in combination with other oligomeric compounds or therapeutics, can be used as tools in differential and/or combinatorial analyses to elucidate expression patterns of a portion or the entire complement of genes expressed within cells and tissues.

As one nonlimiting example, expression patterns within cells or tissues treated with one or more antisense oligomeric compounds are compared to control cells or tissues not treated with antisense oligomeric compounds and the patterns produced are analyzed for differential levels of gene expression as they pertain, for example, to disease association, signaling pathway, cellular localization, expression level, size, structure or function of the genes examined. These analyses can be performed on stimulated or unstimulated cells and in the presence or absence of other compounds and or oligomeric compounds which affect expression patterns.

Examples of methods of gene expression analysis known in the art include DNA arrays or microarrays (Brazma and Vilo, FEBS Lett., 2000, 480, 17-24; Celis, et al., FEBS Lett., 2000, 480, 2-16), SAGE (serial analysis of gene expression)(Madden, et al., Drug Discov. Today, 2000, 5, 415-425), READS (restriction enzyme amplification of digested cDNAs) (Prashar and Weissman, Methods Enzymol., 1999, 303, 258-72), TOGA (total gene expression analysis) (Sutcliffe, et al., Proc. Natl. Acad. Sci. U. S. A., 2000, 97, 1976-81), protein arrays and proteomics (Celis, et al., FEBS Lett., 2000, 480, 2-16; Jungblut, et al., Electrophoresis, 1999, 20, 2100-10), expressed sequence tag (EST) sequencing (Celis, et al., FEBS Lett., 2000, 480, 2-16; Larsson, et al., J. Biotechnol., 2000, 80, 143-57), subtractive RNA fingerprinting (SuRF) (Fuchs, et al., Anal. Biochem., 2000, 286, 91-98; Larson, et al., Cytometry, 2000, 41, 203-208), subtractive cloning, differential display (DD) (Jurecic and Belmont, Curr. Opin. Microbiol., 2000, 3, 316-21), comparative genomic hybridization (Carulli, et al., J. Cell Biochem. Suppl., 1998, 31, 286-96), FISH (fluorescent in situ hybridization) techniques (Going and Gusterson, Eur. J. Cancer, 1999, 35, 1895-904) and mass spectrometry methods (To, Comb. Chem. High Throughput Screen, 2000, 3, 235-41).

The oligomeric compounds of the invention are useful for research and diagnostics, in one aspect because they hybridize to nucleic acids encoding proteins. For example, oligonucleotides that are shown to hybridize with such efficiency and under such conditions as disclosed herein as to be effective protein inhibitors will also be effective primers or probes under conditions favoring gene amplification or detection, respectively. These primers and probes are useful in methods requiring the specific detection of nucleic acid molecules encoding proteins and in the amplification of the nucleic acid molecules for detection or for use in further studies. Hybridization of the antisense oligonucleotides, particularly the primers and probes, of the invention with a nucleic acid can be detected by means known in the art. Such means may include conjugation of an enzyme to the oligonucleotide, radiolabelling of the oligonucleotide or any other suitable detection means. Kits using such detection means for detecting the level of selected proteins in a sample may also be prepared.

The specificity and sensitivity of antisense is also harnessed by those of skill in the art for therapeutic uses. Antisense oligomeric compounds have been employed as therapeutic moieties in the treatment of disease states in animals, including humans. Antisense oligonucleotide drugs, including ribozymes, have been safely and effectively administered to humans and numerous clinical trials are presently underway. It is thus established that antisense oligomeric compounds can be useful therapeutic modalities that can be configured to be useful in treatment regimes for the treatment of cells, tissues and animals, especially humans. For therapeutics, a patient, such as a human, suspected of having a disease or disorder which can be treated by modulating the expression of a gene is treated by administering antisense oligomeric compounds in accordance with this invention. The compounds of the invention can be utilized in pharmaceutical compositions by adding an effective amount of an antisense oligomeric compound to a suitable pharmaceutically acceptable diluent or carrier. Use of the antisense oligomeric compounds and methods of the invention may also be useful prophylactically, e.g., to prevent or delay infection, inflammation or tumor formation, for example. In some embodiments, the patient being treated has been identified as being in need of treatment or has been previously diagnosed as such.

The compositions of the invention may also be admixed, encapsulated, conjugated or otherwise associated with other molecules, molecule structures or mixtures of compounds, as for example, liposomes, receptor-targeted molecules, oral, rectal, topical or other formulations, for assisting in uptake, distribution and/or absorption. Representative U.S. patents that teach the preparation of such uptake, distribution and/or absorption-assisting formulations include, but are not limited to, U.S.: 5,108,921; 5,354,844; 5,416,016; 5,459,127; 5,521,291; 5,543,158; 5,547,932; 5,583,020; 5,591,721; 4,426,330; 4,534,899; 5,013,556; 5,108,921; 5,213,804; 5,227,170; 5,264,221; 5,356,633; 5,395,619; 5,416,016; 5,417,978; 5,462,854; 5,469,854; 5,512,295; 5,527,528; 5,534,259; 5,543,152; 5,556,948; 5,580,575; and 5,595,756.

### Nonlimiting disclosure and incorporation by reference

While certain compounds, compositions and methods described herein have been described with specificity in accordance with certain embodiments, the following examples serve only to illustrate the compounds described herein and are not intended to limit the same. Each of the references, GenBank accession numbers, and the like recited in the present application is incorporated herein by reference in its entirety.

The nucleoside sequences set forth in the sequence listing and Examples, are independent of any modification to a sugar moiety, a monomeric linkage, or a nucleobase. As such, oligomeric compounds defined by a SEQ ID NO may comprise, independently, one or more modifications to a sugar moiety, an internucleoside linkage, or a nucleobase. Oligomeric compounds described by Isis Number (Isis NO.) indicate a combination of nucleobase sequence and one or more modifications to a sugar moiety, an internucleoside linkage, or a nucleobase, as indicated.

### Examples

### General

The sequences listed in the examples have been annotated to indicate where there are modified nucleosides or internucleoside linkages. All non-annotated nucleosides are β-D-ribonucleosides linked by phosphodiester internucleoside linkages. Phosphorothioate internucleoside linkages are indicated by subscript s. Modified nucleosides are indicated by a subscripted letter following the capital letter indicating the nucleoside. In particular, subscript "f" indicates 2'-fluoro; subscript "m" indicates 2'-O-methyl; subscript "1" indicates LNA; subscript "e" indicates 2'-O-methoxyethyl (MOE); subscript "p" indicates 2'-O-propyl; and subscript "t" indicates 4'-thio. For example Uₘ is a modified uridine having a 2'-OCH₃ group. A "d" preceding a nucleoside indicates a deoxynucleoside such as dT which is deoxythymidine. Some of the strands have a 5'-phosphate group designated as "P-". Bolded and italicized "*C*" indicates a 5-methyl C ribonucleoside.

### Example 1: Synthesis of Nucleoside Phosphoramidites

The preparation of nucleoside phosphoramidites is performed following procedures that are extensively illustrated in the art such as but not limited to US Patent 6,426,220 and published PCT WO 02/36743.

### Example 2: Oligonucleotide and oligonucleoside synthesis

The oligomeric compounds used in accordance with this invention may be conveniently and routinely made through the well-known technique of solid phase synthesis. Equipment for such synthesis is sold by several vendors including, for example, Applied Biosystems (Foster City, CA). Any other means for such synthesis known in the art may additionally or alternatively be employed. It is well known to use similar techniques to prepare oligonucleotides such as the phosphorothioates and alkylated derivatives.

Oligonucleotides: Unsubstituted and substituted phosphodiester (P=O) oligonucleotides are synthesized on an automated DNA synthesizer (Applied Biosystems model 394) using standard phosphoramidite chemistry with oxidation by iodine.

Phosphorothioates (P=S) are synthesized similar to phosphodiester oligonucleotides with the following exceptions: thiation was effected by utilizing a 10% w/v solution of 3,H-1,2-benzodithiole-3-one 1,1-dioxide in acetonitrile for the oxidation of the phosphite linkages. The thiation reaction step time was increased to 180 sec and preceded by the normal capping step. After cleavage from the CPG column and deblocking in concentrated ammonium hydroxide at 55°C (12-16 hr), the oligonucleotides were recovered by precipitating with>3 volumes of ethanol from a 1 M NH₄OAc solution. Phosphinate oligonucleotides are prepared as described in U.S. Patent 5,508,270.

Alkyl phosphonate oligonucleotides are prepared as described in U.S. Patent 4,469,863.

3'-Deoxy-3'-methylene phosphonate oligonucleotides are prepared as described in U.S. Patents 5,610,289 or 5,625,050.

Phosphoramidite oligonucleotides are prepared as described in U.S. Patent, 5,256,775 or U.S. Patent 5,366,878.

Alkylphosphonothioate oligonucleotides are prepared as described in published PCT applications PCT/US94/00902 and PCT/US93/06976 (published as WO 94/17093 and WO 94/02499, respectively).

3'-Deoxy-3'-amino phosphoramidate oligonucleotides are prepared as described in U.S. Patent 5,476,925.

Phosphotriester oligonucleotides are prepared as described in U.S. Patent 5,023,243.

Borano phosphate oligonucleotides are prepared as described in U.S. Patents 5,130,302 and 5,177,198.

Oligonucleosides: Methylenemethylimino linked oligonucleosides, also identified as MMI linked oligonucleosides, methylenedimethylhydrazo linked oligonucleosides, also identified as MDH linked oligonucleosides, and methylenecarbonylamino linked oligonucleosides, also identified as amide-3 linked oligonucleosides, and methyleneaminocarbonyl linked oligonucleosides, also identified as amide-4 linked oligonucleosides, as well as mixed backbone oligomeric compounds having, for instance, alternating MMI and P=O or P=S linkages are prepared as described in U.S. Patents 5,378,825, 5,386,023, 5,489,677, 5,602,240 and 5,610,289.

Formacetal and thioformacetal linked oligonucleosides are prepared as described in U.S. Patents 5,264,562 and 5,264,564.

Ethylene oxide linked oligonucleosides are prepared as described in U.S. Patent 5,223,618.

### Example 3: Oligonucleotide Isolation

After cleavage from the controlled pore glass solid support and deblocking in concentrated ammonium hydroxide at 55°C for 12-16 hours, the oligonucleotides or oligonucleosides are recovered by precipitation out of 1 M NH₄OAc with>3 volumes of ethanol. Synthesized oligonucleotides were analyzed by electrospray mass spectroscopy (molecular weight determination) and by capillary gel electrophoresis and judged to be at least 70% full length material. The relative amounts of phosphorothioate and phosphodiester linkages obtained in the synthesis was determined by the ratio of correct molecular weight relative to the -16 amu product (+/-32 +/-48). For some studies oligonucleotides were purified by HPLC, as described by Chiang et al., J. Biol. Chem. 1991, 266, 18162-18171. Results obtained with HPLC-purified material were similar to those obtained with non-HPLC purified material.

### Example 4: Oligonucleotide Synthesis - 96 Well Plate Format

Oligonucleotides can be synthesized via solid phase P(III) phosphoramidite chemistry on an automated synthesizer capable of assembling 96 sequences simultaneously in a 96-well format. Phosphodiester intemucleotide linkages are afforded by oxidation with aqueous iodine. Phosphorothioate intemucleotide linkages are generated by sulfurization utilizing 3,H-1,2 benzodithiole-3-one 1,1 dioxide (Beaucage Reagent) in anhydrous acetonitrile. Standard base-protected beta-cyanoethyl-diiso-propyl phosphoramidites are purchased from commercial vendors (e.g. PE-Applied Biosystems, Foster City, CA, or Pharmacia, Piscataway, NJ). Non-standard nucleosides are synthesized as per standard or patented methods. They are utilized as base protected beta-cyanoethyldiisopropyl phosphoramidites.

Oligonucleotides are cleaved from support and deprotected with concentrated NH₄OH at elevated temperature (55-60°C) for 12-16 hours and the released product then dried *in vacuo.* The dried product is then re-suspended in sterile water to afford a master plate from which all analytical and test plate samples are then diluted utilizing robotic pipettors.

### Example 5: Oligonucleotide Analysis using 96-Well Plate Format

The concentration of oligonucleotide in each well is assessed by dilution of samples and UV absorption spectroscopy. The full-length integrity of the individual products is evaluated by capillary electrophoresis (CE) in either the 96-well format (Beckman P/ACE™ MDQ) or, for individually prepared samples, on a commercial CE apparatus (e.g., Beckman P/ACE™ 5000, ABI 270). Base and backbone composition is confirmed by mass analysis of the oligomeric compounds utilizing electrospray-mass spectroscopy. All assay test plates are diluted from the master plate using single and multi-channel robotic pipettors. Plates are judged to be acceptable if at least 85% of the oligomeric compounds on the plate are at least 85% full length.

### Example 6: Cell culture and oligonucleotide treatment

The effect of oligomeric compounds on target nucleic acid expression can be tested in any of a variety of cell types provided that the target nucleic acid is present at measurable levels. This can be routinely determined using, for example, PCR or Northern blot analysis. Cell lines derived from multiple tissues and species can be obtained from American Type Culture Collection (ATCC, Manassas, VA).

HeLa cells: The human epitheloid carcinoma cell line HeLa was obtained from the American Tissue Type Culture Collection (Manassas, VA). HeLa cells were routinely cultured in DMEM, high glucose (Invitrogen Corporation, Carlsbad, CA) supplemented with 10% fetal bovine serum (Invitrogen Corporation, Carlsbad, CA). Cells were routinely passaged by trypsinization and dilution when they reached 90% confluence. Cells were seeded into 24-well plates (Falcon-Primaria #3846) at a density of 50,000 cells/well or in 96-well plates at a density of 5,000 cells/well for uses including but not limited to oligomeric compound transfection experiments.

When cells reached 65-75% confluency, they were treated with oligonucleotide. Oligonucleotide was mixed with LIPOFECTAMINE™ or with OLIGOFECTAMINE™ Invitrogen Life Technologies, Carlsbad, CA) in Opti-MEM™-1 reduced serum medium (Invitrogen Life Technologies, Carlsbad, CA) to achieve the desired concentration of oligonucleotide and a LIPOFECTAMINE™ or OLIGOFECTAMINE™ concentration of 2.5 or 3 µg/mL per 100 nM oligonucleotide. This transfection mixture was incubated at room temperature for approximately 0.5 hours. For cells grown in 96-well plates, wells were washed once with 100 µL OPTI-MEM™-1 and then treated with 130 µL of the transfection mixture. Cells grown in 24-well plates or other standard tissue culture plates are treated similarly, using appropriate volumes of medium and oligonucleotide. Cells are treated and data are obtained in duplicate or triplicate. After approximately 4-7 hours of treatment at 37°C, the medium containing the transfection mixture was replaced with fresh culture medium. Cells were harvested 16-24 hours after oligonucleotide treatment.

### Example 7: Analysis of oligonucleotide inhibition of a target expression

Antisense modulation of a target expression can be assayed in a variety of ways known in the art. For example, a target mRNA levels can be quantitated by, e.g., Northern blot analysis, competitive polymerase chain reaction (PCR), or real-time PCR. Real-time quantitative PCR is presently desired. RNA analysis can be performed on total cellular RNA or poly(A)+ mRNA. One method of RNA analysis of the present invention is the use of total cellular RNA as described in other examples herein. Methods of RNA isolation are well known in the art. Northern blot analysis is also routine in the art. Real-time quantitative (PCR) can be conveniently accomplished using the commercially available ABI PRISM™ 7600, 7700, or 7900 Sequence Detection System, available from PE-Applied Biosystems, Foster City, CA and used according to manufacturer's instructions.

Protein levels of a target can be quantitated in a variety of ways well known in the art, such as immunoprecipitation, Western blot analysis (immunoblotting), enzyme-linked immunosorbent assay (ELISA) or fluorescence-activated cell sorting (FACS). Antibodies directed to a target can be identified and obtained from a variety of sources, such as the MSRS catalog of antibodies (Aerie Corporation, Birmingham, MI), or can be prepared via conventional monoclonal or polyclonal antibody generation methods well known in the art. Methods for preparation of polyclonal antisera are taught in, for example, Ausubel, F.M. et al., Current Protocols in Molecular Biology, Volume 2, pp. 11.12.1-11.12.9, John Wiley & Sons, Inc., 1997. Preparation of monoclonal antibodies is taught in, for example, Ausubel, F.M. et al., Current Protocols in Molecular Biology, Volume 2, pp. 11.4.1-11.11.5, John Wiley & Sons, Inc., 1997.

Immunoprecipitation methods are standard in the art and can be found at, for example, Ausubel, F.M. et al., Current Protocols in Molecular Biology, Volume 2, pp. 10.16.1-10.16.11, John Wiley & Sons, Inc., 1998. Western blot (immunoblot) analysis is standard in the art and can be found at, for example, Ausubel, F.M. et al., Current Protocols in Molecular Biology, Volume 2, pp. 10.8.1-10.8.21, John Wiley & Sons, Inc., 1997. Enzyme-linked immunosorbent assays (ELISA) are standard in the art and can be found at, for example, Ausubel, F.M. et al., Current Protocols in Molecular Biology, Volume 2, pp. 11.2.1-11.2.22, John Wiley & Sons, Inc., 1991.

### Example 8 : RNA Isolation

### Poly(A)+ mRNA isolation

Poly(A)+ mRNA is isolated according to Miura et al., (Clin. Chem., 1996, 42, 1758-1764). Other methods for poly(A)+ mRNA isolation are routine in the art. Briefly, for cells grown on 96-well plates, growth medium was removed from the cells and each well was washed with 200 µL cold PBS. 60 µL lysis buffer (10 mM Tris-HCl, pH 7.6, 1 mM EDTA, 0.5 M NaCl, 0.5% NP-40, 20 mM vanadyl-ribonucleoside complex) was added to each well, the plate was gently agitated and then incubated at room temperature for five minutes. 55 µL of lysate was transferred to Oligo d(T) coated 96-well plates (AGCT Inc., Irvine CA). Plates were incubated for 60 minutes at room temperature, washed 3 times with 200 µL of wash buffer (10 mM Tris-HCl pH 7.6, 1 mM EDTA, 0.3 M NaCl). After the final wash, the plate was blotted on paper towels to remove excess wash buffer and then air-dried for 5 minutes. 60 µL of elution buffer (5 mM Tris-HCl pH 7.6), preheated to 70°C, was added to each well, the plate was incubated on a 90°C hot plate for 5 minutes, and the eluate was then transferred to a fresh 96-well plate.

Cells grown on 100 mm or other standard plates may be treated similarly, using appropriate volumes of all solutions.

### Total RNA Isolation

Total RNA is isolated using an RNEASY 96™ kit and buffers purchased from Qiagen Inc. (Valencia, CA) following the manufacturer's recommended procedures. Briefly, for cells grown on 96-well plates, growth medium is removed from the cells and each well is washed with 200 µL cold PBS. 150 µL Buffer RLT is added to each well and the plate vigorously agitated for 20 seconds. 150 µL of 70% ethanol is then added to each well and the contents mixed by pipetting three times up and down. The samples are then transferred to the RNEASY 96™ well plate attached to a QIAVAC™ manifold fitted with a waste collection tray and attached to a vacuum source. Vacuum is applied for 1 minute. 500 µL of Buffer RW1 is added to each well of the RNEASY 96™ plate and incubated for 15 minutes and the vacuum is again applied for 1 minute. An additional 500 µL of Buffer RW1 is added to each well of the RNEASY 96™ plate and the vacuum is applied for 2 minutes. 1 mL of Buffer RPE is then added to each well of the RNEASY 96™ plate and the vacuum applied for a period of 90 seconds. The Buffer RPE wash is then repeated and the vacuum is applied for an additional 3 minutes. The plate is then removed from the QIAVAC™ manifold and blotted dry on paper towels. The plate is then re-attached to the QIAVAC™ manifold fitted with a collection tube rack containing 1.2 mL collection tubes. RNA is then eluted by pipetting 140 µL of RNAse free water into each well, incubating 1 minute, and then applying the vacuum for 3 minutes.

The repetitive pipetting and elution steps may be automated using a QIAGEN Bio-Robot 9604 (Qiagen, Inc., Valencia CA). Essentially, after lysing of the cells on the culture plate, the plate is transferred to the robot deck where the pipetting, DNase treatment and elution steps are carried out.

### Example 9: Real-time Quantitative PCR Analysis of target mRNA Levels

Quantitation of a target mRNA levels was accomplished by real-time quantitative PCR using the ABI PRISM™ 7600, 7700, or 7900 Sequence Detection System (PE-Applied Biosystems, Foster City, CA) according to manufacturer's instructions. This is a closed-tube, non-gel-based, fluorescence detection system which allows high-throughput quantitation of polymerase chain reaction (PCR) products in real-time. As opposed to standard PCR in which amplification products are quantitated after the PCR is completed, products in real-time quantitative PCR are quantitated as they accumulate. This is accomplished by including in the PCR reaction an oligonucleotide probe that anneals specifically between the forward and reverse PCR primers, and contains two fluorescent dyes. A reporter dye (e.g., FAM or JOE, obtained from either PE-Applied Biosystems, Foster City, CA, Operon Technologies Inc., Alameda, CA or Integrated DNA Technologies Inc., Coralville, IA) is attached to the 5' end of the probe and a quencher dye (e.g., TAMRA, obtained from either PE-Applied Biosystems, Foster City, CA, Operon Technologies Inc., Alameda, CA or Integrated DNA Technologies Inc., Coralville, IA) is attached to the 3' end of the probe. When the probe and dyes are intact, reporter dye emission is quenched by the proximity of the 3' quencher dye. During amplification, annealing of the probe to the target sequence creates a substrate that can be cleaved by the 5'-exonuclease activity of Taq polymerase. During the extension phase of the PCR amplification cycle, cleavage of the probe by Taq polymerase releases the reporter dye from the remainder of the probe (and hence from the quencher moiety) and a sequence-specific fluorescent signal is generated. With each cycle, additional reporter dye molecules are cleaved from their respective probes, and the fluorescence intensity is monitored at regular intervals by laser optics built into the ABI PRISM™ Sequence Detection System. In each assay, a series of parallel reactions containing serial dilutions of mRNA from untreated control samples generates a standard curve that is used to quantitate the percent inhibition after antisense oligonucleotide treatment of test samples.

Prior to quantitative PCR analysis, primer-probe sets specific to the target gene being measured are evaluated for their ability to be "multiplexed" with a GAPDH amplification reaction. In multiplexing, both the target gene and the internal standard gene GAPDH are amplified concurrently in a single sample. In this analysis, mRNA isolated from untreated cells is serially diluted. Each dilution is amplified in the presence of primer-probe sets specific for GAPDH only, target gene only ("single-plexing"), or both (multiplexing). Following PCR amplification, standard curves of GAPDH and target mRNA signal as a function of dilution are generated from both the single-plexed and multiplexed samples. If both the slope and correlation coefficient of the GAPDH and target signals generated from the multiplexed samples fall within 10% of their corresponding values generated from the single-plexed samples, the primer-probe set specific for that target is deemed multiplexable. Other methods of PCR are also known in the art.

RT and PCR reagents were obtained from Invitrogen Life Technologies (Carlsbad, CA). RT, real-time PCR was carried out by adding 20 µL PCR cocktail (2.5x PCR buffer minus MgCl₂, 6.6 mM MgCl₂, 375 µM each of dATP, dCTP, dCTP and dGTP, 375 nM each of forward primer and reverse primer, 125 nM of probe, 4 Units RNAse inhibitor, 1.25 Units PLATINUM® Taq, 5 Units MuLV reverse transcriptase, and 2.5x ROX dye) to 96-well plates containing 30 µL total RNA solution (20-200 ng). The RT reaction was carried out by incubation for 30 minutes at 48°C. Following a 10 minute incubation at 95°C to activate the PLATINUM® Taq, 40 cycles of a two-step PCR protocol were carried out: 95°C for 15 seconds (denaturation) followed by 60°C for 1.5 minutes (annealing/extension).

Gene target quantities obtained by RT, real-time PCR are normalized using either the expression level of GAPDH, a gene whose expression is constant, or by quantifying total RNA using RIBOGREEN™ (Molecular Probes, Inc. Eugene, OR). GAPDH expression is quantified by real time RT-PCR, by being run simultaneously with the target, multiplexing, or separately. Total RNA is quantified using RiboGreen™ RNA quantification reagent (Molecular Probes, Inc. Eugene, OR). Methods of RNA quantification by RIBOGREEN™ are taught in Jones, L.J., et al, (Analytical Biochemistry, 1998, 265, 368-374).

In this assay, 170 µL of RIBOGREEN™ working reagent (RIBOGREEN™ reagent diluted 1:350 in 10mM Tris-HCl, 1 mM EDTA, pH 7.5) is pipetted into a 96-well plate containing 30 µL purified, cellular RNA. The plate is read in a CytoFluor 4000 (PE Applied Biosystems) with excitation at 485nm and emission at 530nm.

### Example 10: Assay to determining relative activity of certain oligomeric compounds

Six oligomeric compounds were tested for their ability to reduce target mRNA in HeLa cells as described in Example 6. The oligomeric compounds each comprised the same sequence, but differed in their motifs, as indicated in Table 1, below. The 6 compounds were separately assayed with and without their complementary (sense) strands. When sense strand were present, they was full RNA, full phosphodiester. RNA was isolated and analyzed as described in Examples 8-10 and IC50 values for the 6 oligomeric compounds with and without complementary sense strands are in Table 1.

**Table 1: Reduction of target RNA in HeLa cells using single and double stranded oligomeric compounds.**

| Isis # | Sequence (antisense) 5'-3' | IC₅₀ of double strand duplex (nM) | IC₅₀ of single strand (nM) | SEQ ID |
|---|---|---|---|---|
| 341391 | UₒUₒGₒUₒCₒUₒCₒUₒGₒGₒUₒCₒCₒUₒUₒAₒCₒUₒU | 0.3 | >100 | 4 |
| 303912 | PoUₛUₛGₛUₛCₛUₛCₛUₛGₛGₛUₛCₛCₛUₛUₛAₛCₛUₛU | 1.0 | 47 | 4 |
| 359455 | UₒUₒGₒUₒCₒUₒCₒUₒGₒGₒUₒCₛCₛUₛUₛAₛCₛUₛU | 0.25 | 39 | 4 |
| 359456 | UₒUₒGₒUₒCₒUₒCₒUₒGₒGₒUₛCₛCₛUₛUₛAₛCₛUₛU | 0.3 | >200 | 4 |
| 359458 | UₛUₛGₛUₛCₛUₛCₛUₛGₛGₛUₛCₛCₛUₛUₛAₛCₛUₛU | 1.0 | >200 | 4 |
| 386187 | PoU_{fo}U_{fo}G_{fo}U_{fo}C_{fo}U_{fo}C_{fo}U_{fo}G_{fo}G_{fo}U_{fo}C_{fs}C_{fs}U_{fs}U_{fs} A_{fs}C_{fs}U_{fs}U_{f} | 0.01 | 0.43 | 4 |

| | | | | |
|---|---|---|---|---|
| N = RNA; o = phosphodiester; s = phosphorothioate; f = 2'-Fluoro; Po = phosphate cap As indicated in Table 1, all of the oligomeric compounds were effective at reducing target mRNA when paired with a sense strand and contacted with Hela cells in the presence of LIPFECTIN. The full 2'F, with 7 phosphorothioate linkages at the 3' end and a 5'-phosphate had good activity when used as a single-strand. | | | | |

Various modifications of the invention, in addition to those described herein, will be apparent to those skilled in the art from the foregoing description. Such modifications are also intended to fall within the scope of the appended claims. Each reference (including, but not limited to, journal articles, U.S. and non-U.S. patents, patent application publications, international patent application publications, gene bank accession numbers, and the like) cited in the present application is incorporated herein by reference in its entirety.

### Embodiments of the invention:

1. An oligomeric compound comprising a single-stranded oligonucleotide consisting of 17-24 linked nucleosides wherein each nucleoside comprises a 2'-F modification;
   each of the 6 to 9 3'-most internucleoside linkages is a phosphorothioate linkage and each of the other internucleoside linkages is a phosphodiester linkage.
2. The oligomeric compound of embodiment 1 wherein each of the 7 3'-most internucleoside linkages is a phosphorothioate linkage and the other linkages and each of the other internucleoside linkages is a phosphodiester linkage.
3. The oligomeric compound of embodiment 1 wherein each of the 8 3'-most internucleoside linkages is a phosphorothioate linkage and the other linkages and each of the other internucleoside linkages is a phosphodiester linkage.
4. The oligomeric compound of any of embodiments 1-3 comprising one or more terminal phosphate, conjugate group or capping group.
5. The oligomeric compound of embodiment 4 wherein the oligomeric compound comprises a phosphate on the 5'-end of the oligonucleotide.
6. The oligomeric compound of any of the embodiments 1-5 comprising 1-3 non-hybridizing terminal nucleosides on either the 3' end or the 5' end, or on both the 3' end and the 5' end of the oligonucleotide.
7. A pharmaceutical composition comprising an oligomeric compound according to any of embodiments 1-6 and a pharmaceutically acceptable carrier.
8. A method of inhibiting protein expression in a cell by contacting the cell with an oligomeric compound according to any of embodiments 1-6.
9. The method of embodiment 8 wherein the cell is in an animal.
10. The method of embodiment 9 wherein the animal is a human.
11. A method of affecting cleavage of a target RNA via the RNAi pathway in cells or tissues by contacting said cells or tissues with an oligonucleotide of according to any of embodiments 1-6.
12. The method of embodiment 11 wherein the cell is in an animal.
13. The method of embodiment 11 wherein the animal is a human.

## Claims

1. An oligomeric compound comprising a single-stranded oligonucleotide consisting of 17-24 linked nucleosides wherein a plurality of the nucleosides are modified nucleosides, each of the 6-13 3'-most internucleoside linkages is a phosphorothioate linkage and each of the other internucleoside linkages is a phosphodiester linkage.

2. The oligomeric compound of claim 1, wherein each nucleoside of the oligonucleotide is a 2'-F nucleoside.

3. The oligomeric compound of claim 1 or claim 2, comprising a single-stranded oligonucleotide consisting of 17-24 linked nucleosides wherein each nucleoside comprises a 2'-F modification; each of the 6 to 9 3'-most internucleoside linkages is a phosphorothioate linkage and each of the other internucleoside linkages is a phosphodiester linkage.

4. The oligomeric compound of any one of claims 1-3 wherein each of the 7 3'-most internucleoside linkages is a phosphorothioate linkage and the other linkages and each of the other internucleoside linkages is a phosphodiester linkage.

5. The oligomeric compound of any one of claims 1-3 wherein each of the 8 3'-most internucleoside linkages is a phosphorothioate linkage and the other linkages and each of the other internucleoside linkages is a phosphodiester linkage.

6. The oligomeric compound of any of claims 1-5 comprising one or more terminal phosphate, conjugate group or capping group.

7. The oligomeric compound of claim 6 wherein the oligomeric compound comprises a phosphate on the 5'-end of the oligonucleotide.

8. The oligomeric compound of any of the claims 1-7 comprising 1-3 non-hybridizing terminal nucleosides on either the 3' end or the 5' end, or on both the 3' end and the 5' end of the oligonucleotide.

9. A pharmaceutical composition comprising an oligomeric compound according to any of claims 1-8 and a pharmaceutically acceptable carrier.

10. A method of inhibiting protein expression in a cell by contacting the cell with an oligomeric compound according to any of claims 1-8.

11. The method of claim 10 wherein the cell is in an animal.

12. The method of claim 11 wherein the animal is a human.

13. A method of affecting cleavage of a target RNA via the RNAi pathway in cells or tissues by contacting said cells or tissues with an oligonucleotide of according to any of claims 1-8.

14. The method of claim 13 wherein the cell is in an animal.

15. The method of claim 13 wherein the animal is a human.
